## Europäisches Patentamt

## European Patent Office

## Office européen des brevets

(19)

(11) Publication number: **0 195 490 B1**

# EUROPEAN PATENT SPECIFICATION

(12)

(45) Date of publication of the patent specification: 02.08.89

(51) Int. Cl.⁴: **F16F 9/02, F16F 9/54, A61B 6/00**

(21) Application number: **86200466.0**

(22) Date of filing: **20.03.86**

(54) Adjustable gas spring.

(30) Priority: **21.03.85 NL 8500831**

(43) Date of publication of application:
**24.09.86 Bulletin 86/39**

(45) Publication of the grant of the patent:
**02.08.89 Bulletin 89/31**

(84) Designated Contracting States:
**DE FR GB IT NL**

(56) References cited:
**DE-A- 1 429 543**
**DE-A- 1 554 256**
**DE-A- 3 137 193**
**FR-A- 841 174**
**FR-A- 2 532 386**
**GB-A- 215 933**
**US-A- 1 513 971**
**US-A- 1 606 467**
**US-A- 2 939 697**

(73) Proprietor: **Holland Hellas Hydrauliek & Pneumatiek B.V., Diamantstraat 33, NL-7554 TA Hengelo(NL)**

(72) Inventor: **Tiehuis, Joseph Hermanus Maria, Drilscholtenstraat 23, NI-7556 NR Hengelo(NL)**

(74) Representative: **Cammel, Willem Frans et al, OCTROOIBUREAU ARNOLD & SIEDSMA Sweelinckplein 1, NL-2517 GK The Hague(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to an adjustable spring comprising a cylinder filled with a medium under pressure, a piston operative as a first displacement member axially movable in said cylinder in sealing relationship therewith, said piston being provided with a narrow passage and connected with a piston rod extending outside said cylinder in coaxial and sealing relationship therewith, a second displacement member movable axially in said cylinder in sealing relationship therewith and having a position that can be adjusted for adjusting the operative volume of said cylinder, and thereby the slide force, and a rod extending coaxially with and outside the cylinder, said rod being provided with thread, running through an end wall of said cylinder which is provided with a correspondingly threaded aperture and being connected with the second displacement member, such that by rotation of said rod relative to said cylinder the axial position of said second displacement member can be adjusted.

Such an adjustable spring is known from e.g. DE-A 1 429 543.

Relative to this prior art specification it is a purpose of the invention to design an adjustable spring in a manner such that the spring can be adjusted whilst nevertheless maintaining the effective length of the spring.

To this end the adjustable spring according to the invention is characterized in that at the end of the piston rod a first attachment element is arranged, and the free end of the threaded rod extending outside the cylinder is provided with the same thread as the part co-operating with the threaded aperture in the end wall of the cylinder, said second thread co-operating with a corresponding thread aperture in a second attachment element, such that, with rotation of said threaded rod relative to the cylinder and said second attachment element, the relative positions of the first and the second attachment elements remain unaltered.

A very simple embodiment is one in which the second displacement member is connected exclusively with the rod since as a result of the pressure of the medium in the cylinder this displacement member is pressed against the free end and thereof.

In a practical embodiment the spring according to the invention is characterized in that in the area of the transition between the one thread and the other thread, the threaded rod carries a contact member to apply, if necessary under application of a tool, a turning moment to said threaded rod.

The invention will now be elucidated on the basis of a drawing of a random embodiment, in which:

Fig. 1 shows a cross section through a preferred embodiment; and
Fig. 2 shows a perspective view of a device in which the gas spring according to the invention is applied.

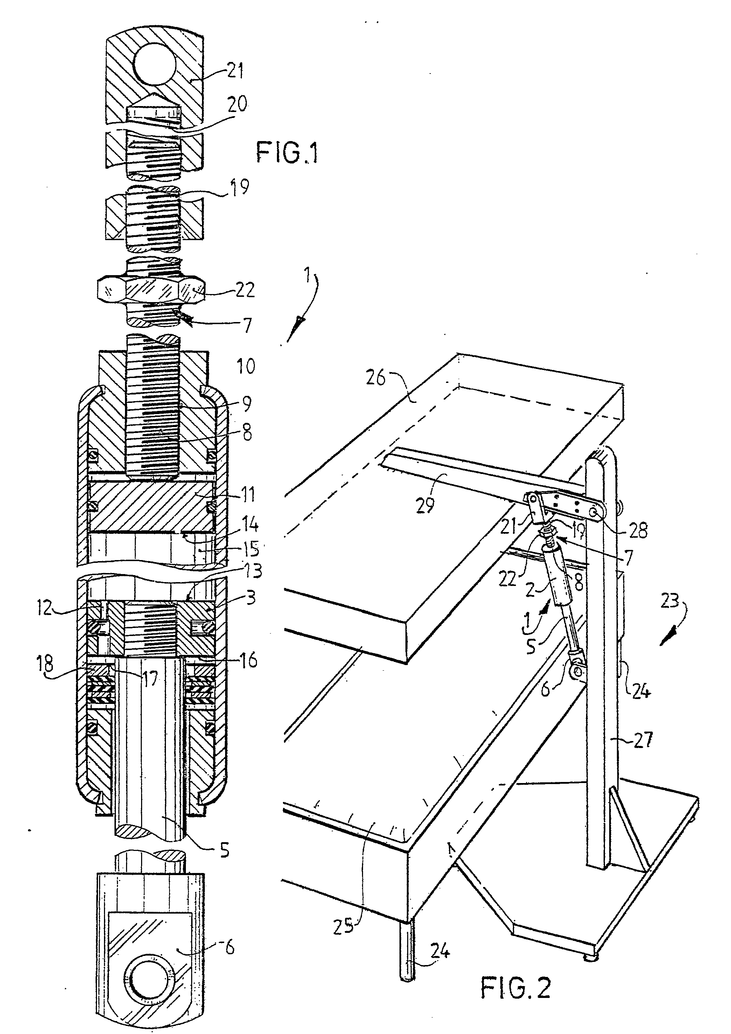

Fig. 1 shows a gas spring 1, comprising a cylinder 2 filled with gas, a piston 3 that is axially movable in the cylinder and sealing it, the piston being provided with a narrow passage 12 and having a piston rod 5 extending outside cylinder 2 coaxially with it and sealing it by means of sealing rings 4, at the end of which rod a fixing eye 6 is arranged, and a rod 7 extending on the other side outside cylinder 2 and coaxially with it. This rod 7 is provided with thread 8. It runs through an end wall 10 of cylinder 2 provided with a corresponding threaded aperture 9 and rests against a second piston 11 such that, as a result of rotation of rod 7 relative to cylinder 2, the axial position of the second piston 11 can be adjusted.

Once the second piston 11 is fixed in a selected position, the first piston 3 can then be moved axially between two extreme positions, namely, a compressed position, in which the faces 13 and 14 directed towards each other of respectively the first piston 3 and the second piston 11 lie against each other, and a position in which gas spring 1 has its greatest length, and in which the faces 16 and 17 directed towards each other of respectively the first piston 3 and the second end wall 18 lie against each other.

By adjusting the axial position of the second piston 11 the active volume, i.e. the space that is bounded by end surfaces 14 and 17 and the inner surface of cylinder 2, can be determined.

The free end of rod 7 is provided with screw-thread 19, which has the same pitch as screw-thread 8. Screw-thread 19 co-operates with a corresponding threaded aperture 20 in a second fixing eye 21 such that, with rotation of rod 7, the interval between the first fixing eye 6 and the second fixing eye 21 remains unaltered.

In the area of transition between screw-thread 8 and screw-thread 19 rod 7 carries a nut 22 for applying, with the aid of an open-ended spanner or other tool, a turning moment to rod 7 for adjustment of the axial position of the second piston 11 and thereby of the spring characteristic of gas spring 1.

Fig. 2 shows a device 23, namely an application of the gas spring 1 according to the invention within the framework of a sunbed. This device comprises a fixed table 25 supported by legs 24. A frame 27 bears an upper plate 26 that can pivot round a hinge 28 relative to a horizontal axis, in which plate radiation sources generating ultraviolet rays are present, this plate 26 being movable between two extreme positions, namely, a raised position and a lowered position or operating position. Plate 26 on the hinge is supported by two arms 29. One of these arms 29 is coupled with frame 27 by means of gas spring 1 as according to fig. 1. The position shown in fig. 2, the operating position, is now determined by the equilibrium of force between the weight of plate 26 and the spring characteristic of gas spring 1, particularly its slide force. This can be adapted to the weight of plate 26 by rotating nut 22 as required.

## Claims

1. Adjustable spring (1) comprising a cylinder (2) filled with a medium under pressure, a piston (3) operative as a first displacement member axially movable in said cylinder (2) in sealing relationship therewith, said piston (3) being provided with a narrow passage (12) and connected with a piston rod (5) extending outside said cylinder (2) in coaxial and sealing relationship therewith, a second displacement member (11) movable axially in said cylinder (2) in sealing relationship therewith and having a position that can be adjusted for adjusting the operative volume of said cylinder (2), and thereby the slide force, and a rod (7) extending coaxially with and outside the cylinder (2), said rod (7) being provided with thread (19), running through an end wall (10) of said cylinder (2) which is provided with a correspondingly threaded aperture (9) and being connected with the second displacement member (11), such that by rotation of said rod (7) relative to said cylinder (2) the axial position of said second displacement member (11) can be adjusted, characterized in that at the end of the piston rod (5) a first attachment element (6) is arranged, and the free end of the threaded rod (7) extending outside the cylinder (2) is provided with the same thread (19) as the part co-operating with the threaded aperture (9) in the end wall (10) of the cylinder (2), said second thread (19) co-operating with a corresponding thread aperture (20) in a second attachment element (21), such that, with rotation of said threaded rod (7) relative to the cylinder (2) and said second attachment element (21), the relative positions of the first and the second attachment elements (6; 21) remain unaltered.

2. Adjustable spring as claimed in claim 1, characterized in that the second displacement member (11) is connected exclusively with the rod (7), since as a result of the medium pressure in the cylinder (2), said displacement member (11) is pressed against the free end thereof.

3. Adjustable spring as claimed in claims 1 or 2, characterized in that, in the area of the transition between the one thread (8) and the other thread (19), the threaded rod (7) carries a contact member (22) to apply, if necessary under application of a tool, a turning moment to said threaded rod (7).

**Patentansprüche**

1. Einstellbare Feder (1) mit einem Zylinder (2), der mit einem Druckmedium gefüllt ist, einem Kolben (3), der als ein erstes Stellglied wirksam ist, das in dem Zylinder (2), in Dichtebeziehung mit diesem, axial bewegbar ist, wobei der Kolben (3) mit einem engen Durchgang (12) versehen ist und mit einer Kolbenstange (5) verbunden ist, die sich aus dem Zylinder (2), in Koaxial- und Dichtebeziehung mit diesem, heraus erstreckt, einem zweiten Stellglied (11), das in dem Zylinder (2), in Dichtebeziehung mit diesem, axial bewegbar ist und eine Position hat, die verstellt werden kann, um das Wirkvolumen des Zylinders (2) und dadurch die Schubkraft einzustellen, und eine Stange (7), die sich koaxial zum Zylinder (2) und aus diesem heraus erstreckt, wobei die Stange (7), die mit einem Gewinde (19) versehen ist, durch eine Stirnwand (10) des Zylinders (2) verläuft, welche mit einer mit einem entsprechenden Gewinde versehenen Öffnung (9) versehen ist und mit dem zweiten Stellglied so verbunden ist, daß bei Drehung der Stange (7), relativ zum Zylinder (2), die axiale Position des zweiten Stellglieds (11) verstellt werden kann, dadurch gekennzeichnet, daß an dem Ende der Kolbenstange (5) ein erstes Befestigungselement (6) angeordnet ist, und das freie Ende der aus dem Zylinder (2) herausragenden Gewindestange (7) mit dem gleichen Gewinde (19) versehen ist, wie das mit der Gewindeöffnung (9) in der Stirnwand (10) des Zylinders (2) zusammenwirkende Teil, wobei das zweite Gewinde (19) mit einer entsprechenden Gewindeöffnung (20) in einem zweiten Befestigungselement (21) so zusammenwirkt, daß bei Drehung der Gewindestange (7) relativ zum Zylinder (2) und zum zweiten Befestigungselement (21) die relativen Stellungen des ersten und des zweiten Befestigungselements (6; 21) unverändert bleiben.

2. Einstellbare Feder nach Anspruch 1, dadurch gekennzeichnet, daß nur das zweite Stellglied (11) mit der Stange (7) verbunden ist, da, als Folge des Mediumdruckes im Zylinder (2), das Stellglied (11) gegen deren freies Ende gedrückt wird.

3. Einstellbare Feder nach Anspruch 1 oder 2, dadurch gekennzeichnet, daß in dem Bereich des Übergangs zwischen dem einen Gewinde (8) und dem anderen Gewinde (19) die Gewindestange (7) ein Kontaktglied (22) trägt, um, falls notwendig, unter Verwendung eines Werkzeugs, ein Drehmoment an die Gewindestange (7) anzusetzen.

**Revendications**

1. Ressort réglable (1) comprenant un cylindre (2) rempli d'un fluide sous pression, un piston (3) fonctionnant comme un premier organe à déplacement, mobile axialement dans le cylindre (2) en relation étanche avec ce dernier, le piston (3) étant pourvu d'un passage étroit (12) et étant lié à une tige de piston (5) qui s'étend à l'extérieur du cylindre (2), coaxialement à celui-ci et en relation étanche avec lui, un second organe à déplacement (11) mobile axialement dans le cylindre (2) en relation étanche avec ce dernier et ayant une position qui peut être régler pour régler le volume utile dudit cylindre (2), et par conséquent la force de glissement, et une tige (7) s'étendant coaxialement au cylindre (2) et à l'extérieur de ce dernier, la tige (7) étant pourvue d'un filetage (19), traversant une paroi d'extrémité (10) du cylindre (2) qui comporte une ouverture (9) taraudée de façon correspondante et étant liée au second organe à déplacement (11), de telle sorte que la position axiale de ce second organe à déplacement (11) peut être réglée par rotation de ladite tige (7) par rapport au cylindre (2), caractérisé en ce qu'un premier organe de fixation (6) est disposé à l'extrémité de la tige de piston (6), et en ce que l'extrémité libre de la tige filetée (7), s'étendant à l'extérieur du cylindre (2), est pourvue du même filetage (19) que la partie coopérant avec l'ouverture taraudée (9) ménagée dans la paroi d'extrémité (10) du cylindre (2), ce deuxième filetage (19) coopérant avec un trou taraudé correspondant (20) d'un second organe de fixation (21), de telle sorte que, lors de la rotation de la tige filetée (7) par rapport au cylindre (2) et a second organe de fixation (21), les positions relatives des premier et second organes de fixation (6; 21) restent inchangées.

2. Ressort réglable selon la revendication 1, caractérisé en ce que le second organe à déplacement (11) est lié avec la tige (7) exclusivement par l'effet de la pression du fluide dans le cylindre (7), qui pousse cet organe à déplacement (11) contre l'extrémité libre de ladite tige.

3. Ressort réglable selon les revendications 1 ou 2, caractérisé en ce que, dans la zone de transition entre l'un des filetages (8) et l'autre filetage (19), la tige filetée (7) porte un organe de manœuvre (22) prévu pour appliquer un couple de rotation à cette tige filetée (7), si nécessaire à l'aide d'un outil.

FIG.1

FIG.2